(19) Europäisches Patentamt
European Patent Office
Office européen des brevets

(11) Veröffentlichungsnummer : **0 395 984 B1**

# (12) EUROPÄISCHE PATENTSCHRIFT

(45) Veröffentlichungstag der Patentschrift : **30.12.92 Patentblatt 92/53**

(51) Int. Cl.$^5$ : **C07C 253/30**

(21) Anmeldenummer : **90107707.3**

(22) Anmeldetag : **24.04.90**

## (54) Verfahren zur Herstellung von 2-Cyan-3,3-diarylacrylsäureestern.

(30) Priorität : **29.04.89 DE 3914382**

(43) Veröffentlichungstag der Anmeldung : **07.11.90 Patentblatt 90/45**

(45) Bekanntmachung des Hinweises auf die Patenterteilung : **30.12.92 Patentblatt 92/53**

(84) Benannte Vertragsstaaten : **CH DE FR GB IT LI NL**

(56) Entgegenhaltungen :
**DE-A- 2 524 930**
**DE-A- 2 525 026**

(56) Entgegenhaltungen :
**CHEMICAL ABTRACTS Band 100, 1984, Seite 636, Abstract Nr. 103185b, Columbus, Ohio, US; & SU - A - 1051075**
**CHEMICAL ABSTRACTS, Band 93, 25. August - 1. September 1980, Seite 20, Abstract Nr. 72584m, Columbus, Ohio, US; & SU - A- 726086**

(73) Patentinhaber : **BASF Aktiengesellschaft**
**Carl-Bosch-Strasse 38**
**W-6700 Ludwigshafen (DE)**

(72) Erfinder : **Spang, Peter, Dr.**
**Zur Audell 43**
**W-6670 St. Ingbert (DE)**
Erfinder : **Neumann, Peter, Dr.**
**Poststrasse 28**
**W-6800 Mannheim 31 (DE)**

EP 0 395 984 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft ein neues Verfahren zur Herstellung von 2-Cyan-3,3-diarylacrylsäureestern der allgemeinen Formel I

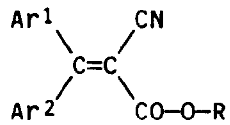

(I)

in der $Ar^1$ und $Ar^2$ aromatische Reste und R einen aliphatischen Rest mit mehr als 2 C-Atomen bedeutet.

Es ist allgemein bekannt (siehe z.B. US-A 3 215 724), die als UV-Absorber in organischen Substanzen dienenden Verbindungen vom Typ I durch Umsetzung eines Diarylketons mit einem Cyanessigester gemäß dem Reaktionsschema

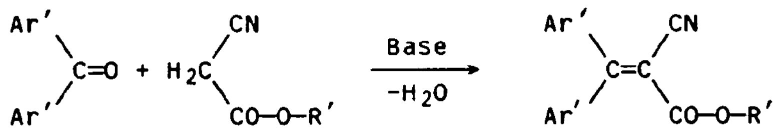


(Ar'= Aryl; R' = organischer Rest)

herzustellen.

Nachteilig bei dieser Darstellungsweise ist jedoch, daß man meistens verfärbte Verfahrensprodukte erhält, die nur mit großem technischen Aufwand zu reinigen sind.

Der Erfindung lag daher die Aufgabe zugrunde, diesem Mangel abzuhelfen.

Demgemäß wurde ein Verfahren zur Herstellung von 2-Cyan-3,3-diarylacrylsäureestern der Formel I gefunden, welches dadurch gekennzeichnet ist, daß man einen 2-Cyan-3,3-diarylacrylsäureester der allgemeinen Formel II

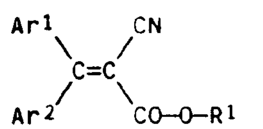

(II)

in der $R^1$ für die Methyl- oder Ethylgruppe steht, in Gegenwart eines basischen Katalysators mit einem Alkohol III

R-OH (III)

unter laufender Entfernung des hierbei entstehenden Alkohols $R^1$-OH umsetzt.

Die als Ausgangsstoffe dienenden Methyl- bzw. Ethylester II der 2-Cyan-3,3-diarylacrylsäuren sind bekannt bzw. auf bekannte Weise in reiner Form durch Umsetzung einer Benzophenonkomponente mit einem aktivierten Cyanessigester (z.B. US 3 149 148) erhältlich.

Im Hinblick auf die Eigenschaften der herzustellenden UV-Absorber I geht man vorzugsweise von solchen Verbindungen II aus, in denen die Reste $Ar^1$ und $AR^2$ folgende Bedeutung haben:

- isocyclische unsubstituierte Arylgruppen wie die α- und β-Napthylgruppe und vor allem die Phenylgruppe;
- substituierte Arylgruppen, insbesondere substituierte Phenylgruppen, mit vorzugsweise bis zu drei der folgenden Substituenten:
- $C_1$-$C_{18}$-Alkylgruppen, insbesondere Methyl- und Ethylgruppen,
- $C_1$-$C_{18}$-Alkoxygruppen, insbesondere die Methoxygruppe,
- Halogen, vorzugsweise Chlor,
- Aryl enthaltende Reste, bevorzugt Benzyl, Ethylphenyl und Phenoxy,
- Stickstoff enthaltende Gruppen, bevorzugt Cyano.

Reste R in den erfindungsgemäß herzustellenden Verbindungen sind vorzugsweise

- $C_4$-$C_{18}$-Alkylgruppen, besonders die 2-Ethylhexyl- und die n-Octylgruppe;

- $C_4$-$C_{18}$-Mono- und Poly-Alkoxyalkylgruppen, bevorzugt die Ethoxyethylgruppe und $CH_3$-O-$CH_2CH_2$-O-$CH_2CH_2$.

Die Reste R können ihrerseits weitere Substituenten wie Aryl-, Cyano-, tertiäre Amino-, Halogen-, Carbonyl- und Aldehydgruppen tragen.

Für die Umesterung geeignete Katalysatoren sind

- basische Alkali- und Erdalkalisalze, bevorzugt solche, die weder in den Edukten noch in den Produkten löslich sind und sich nach Reaktionsende leicht abtrennen lassen, besonders bevorzugt Natrium-, Kalium- oder Calciumcarbonat oder Natriumhydrogencarbonat;
- Erdalkalioxide, bevorzugt Calcium- oder Magnesiumoxid und
- basische Zeolithe.

Die Menge der Katalysatoren beträgt im allgemeinen 1-80 mol-%, bevorzugt 5-50 mol-%, der Menge des eingesetzten Esters II.

Die Menge an Alkohol der Formel III muß mindestens äquimolar sein zur eingesetzten Menge an 2-Cyan-3,3-diarylacrylsäureester der Formel II. Bevorzugt werden Mengen von 200-500 mol-% des Alkohols verwendet.

Die Entfernung des gebildeten Alkohols $R^1$-OH erfolgt destillativ, bevorzugt mit Hilfe eines Gasstromes aus einem inerten Gas wie vorzugsweise Argon oder Stickstoff. Die verwendete Menge an Inertgas beträgt bevorzugt 20-80 l/h.

Vorzugsweise arbeitet man bei Temperaturen von 90-180°C, besonders von 120-160°C.

Besondere Bedingungen bezüglich des Druckes sind nicht erforderlich; im allgemeinen nimmt man die Umsetzung bei Atmosphärendruck vor.

Als Lösungsmittel können inerte höher siedende Verbindungen wie Xylole, aber auch Toluol oder Gemische der eingesetzten Alkohole R-OH mit flüssigen, kurzkettigen Alkanen wie Hexan und Heptan, eingesetzt werden. Bevorzugt arbeitet man lösungsmittelfrei in dem eingesetzten Alkohol R-OH.

Das erfindungsgemäße Verfahren kann sowohl diskontinuierlich als auch kontinuierlich durchgeführt werden. Bei der kontinuierlichen Arbeitsweise leitet man die Reaktionspartner vorzugsweise über ein Festbett aus einer unlöslichen Base.

Die Aufarbeitung des Reaktionsgemisches erfolgt wie üblich, so daß sich nähere Ausführungen hierüber erübrigen.

Die Verfahrensprodukte I werden in hoher Reinheit mit fast quantitativen Ausbeuten ohne Verfärbung erhalten.

Beispiel 1

Herstellung von 2-Cyan-3,3-diphenylacrylsäure-(2-ethyl)hexylester

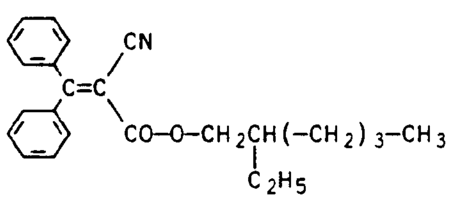

138,5 g (0,5 mol) 2-Cyan-3,3-diphenylacrylsäureethylester und 196 g (1,5 mol) 2-Ethylhexanol wurden bei 130°C in Gegenwart von 5 g (0,05 mol) Natriumcarbonat unter destillativer Entfernung des gebildeten Ethanols, die durch einen Stickstoffstrom unterstützt wurde (ca. 22 l/h), miteinander umgesetzt. Nach etwa einer Stunde war die Umesterung beendet und die Reaktionslösung wurde heiß von dem Natriumcarbonat abfiltriert. Der 2-Ethylhexylester der 2-Cyan-3,3-diphenylacrylsäure fiel bereits in sehr reiner Form an.

Die Reinigung mittels Dünnschicht-Verdampfer lieferte den Ester in Form eines hellgelben Öls in 97 %iger Ausbeute (Reinheit: 99,8 % nach GC-Analyse).

Das gleiche Ergebnis wurde mit Calciumcarbonat sowie mit Magnesiumoxid als Basen nach 2- bzw. 3-stündiger Reaktionsdauer erzielt.

Beispiel 2

Herstellung von 2-Cyan-3,3-diphenylacrylsäureoctylester

Diese Verbindung wurde analog Beispiel 1 hergestellt; hellgelbes Öl, Ausbeute 96 %.

Beispiel 3

Herstellung von 2-Cyan-3,3-diphenylacrylsäure-iso-decyl-ester

Ein Gemisch aus primären iso-Decanolen wurde analog Beispiel 1 zu dem entsprechenden Estergemisch umgesetzt; hellgelbes Öl, Ausbeute 88 %.

## Patentansprüche

1. Verfahren zur Herstellung von 2-Cyan-3,3-diarylacrylsäureestern der allgemeinen Formel I

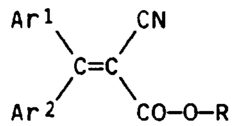

(I)

in der $Ar^1$ und $Ar^2$ aromatische Reste und R einen aliphatischen Rest mit 3 bis 30 C-Atomen bedeuten, dadurch gekennzeichnet, daß man einen 2-Cyan-3,3-diarylacrylsäureester der allgemeinen Formel II

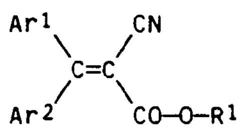

(II)

in der $R^1$ für die Methyl- oder Ethylgruppe steht, in Gegenwart eines basischen Katalysators mit einem Alkohol III

R-OH (III)

unter laufender Entfernung des hierbei entstehenden Alkohols $R^1$-OH umsetzt.

2. Verfahren nach Anspruch 1, dadurch gekennzeichnet, daß man die laufende Entfernung des Alkohols $R^1$-OH mittels eines Inertgasstromes vornimmt.

3. Verfahren nach den Ansprüchen 1 oder 2, dadurch gekennzeichnet, daß man es auf die Herstellung von Verbindungen I anwendet, in denen $Ar^1$ und $Ar^2$ jeweils die Phenylgruppe bedeuten.

4. Verfahren nach den Ansprüchen 1 bis 3, dadurch gekennzeichnet, daß man es auf die Herstellung von Verbindungen I anwendet, in denen R eine $C_8$-$C_{12}$-Alkylgruppe bedeutet.

## Claims

1. A process for preparing 2-cyano-3,3-diarylacrylic esters of the formula I

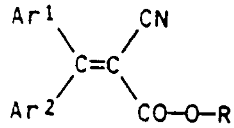

(I)

where $Ar^1$ and $Ar^2$ are aromatic radicals and R is an aliphatic radical with from 3 to 30 C atoms, which comprises reacting a 2-cyano-3,3-diarylacrylic ester of the formula II

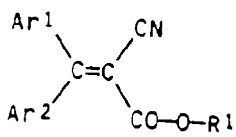

(II)

where $R^1$ is methyl or ethyl, in the presence of a basic catalyst with an alcohol III

R-OH (III)

with continuous removal of the alcohol $R^1$-OH produced thereby.

2. A process as claimed in claim 1, wherein the continuous removal of the alcohol $R^1$-OH is carried out by means of a stream of inert gas.

3. A process as claimed in claims 1 or 2, which is used to prepare compounds I where $Ar^1$ and $Ar^2$ are each phenyl.

4. A process as claimed in claims 1 to 3, which is used to prepare compounds I where R is $C_8$-$C_{12}$-alkyl.

**Revendications**

1. Procédé de préparation de 2-cyano-3,3-diarylacrylates de formule générale I

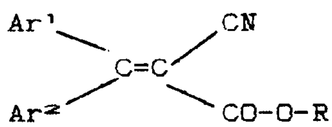

(I)

dans laquelle $Ar^1$ et $Ar^2$ représentent des restes aromatiques et R représente un reste aliphatique à 3-30 atomes de carbone, caractérisé en ce qu'on fait réagir un 2-cyano-3,3-diarylacrylate de formule générale II

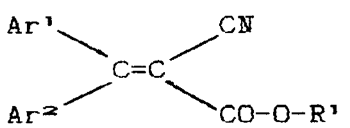

(II)

dans laquelle R′ est mis pour le groupement méthyle ou éthyle, en présence d'un catalyseur basique, avec un alcool III

R-OH (III)

avec élimination continue de l'alcool R′-OH qui se forme au cours de la réaction.

2. Procédé selon la revendication 1, caractérisé en ce qu'on effectue l'élimination continue de l'alcool R′-OH au moyen d'un courant de gaz inerte.

3. Procédé selon la revendication 1 ou 2, caractérisé en ce qu'on l'applique à la préparation de composés I dans lesquels $Ar^1$ et $Ar^2$ représentent chacun le groupement phényle.

4. Procédé selon l'une quelconque des revendications 1 à 3, caractérisé en ce qu'on l'applique à la préparation de composés I dans lequels R représente un groupement alkyle en $C_8$-$C_{12}$.